# FASCICULE DE BREVET EUROPEEN

(11) **EP 0 552 579 B1**
(45) Date de publication et mention de la délivrance du brevet: **03.01.1996**
(21) Numéro de dépôt: 92440007.0
(22) Date de dépôt: 22.01.1992
(51) Int. Cl.: A61F 2/12

(54) **Implants prothétiques pour chirurgie esthétique**
Prosthetisches Implantat für kosmetische Chirurgie
Prosthetic implants for plastic surgery

(43) Date de publication de la demande: 28.07.1993
(73) Titulaire: Muller, Guy-Henri, F-67000 Strasbourg (FR)
(72) Inventeur: Muller, Guy-Henri, F-67000 Strasbourg (FR)
(74) Mandataire: Bossard, Jacques-René

(56) Documents cités:
- GB-A- 2 047 101
- US-A- 4 157 085
- BRITISH J. OF PLASTIC SURGERY vol. 23, 1970, pages 58 - 62 Y. MUTOU 'AUGMENTATION MAMMAPLASTY WITH THE AKIYAMA PROSTHESIS'

## Description

La présente invention concerne les implants prothétiques, notamment les implants mammaires, utilisés en chirurgie plastique, esthétique et réparatrice pour réparer des irrégularités du corps humains, en augmenter le volume et/ou en modifier les contours.

De tels implants ont fait l'objet de nombreuses études et un mode de constitution particulièrement intéressant est décrit dans FR-A-2 630 637 du Déposant, selon lequel l'implant est constitué par une enveloppe mince en silicone remplie d'un grand nombre de petits volumes individuels fermés, délimités chacun par une membrane également en silicone et remplis de sérum physiologique.

La mise en oeuvre de ces implants a cependant rencontré une difficulté, résultant du fait que lesdits volumes individuels doivent subir une stérilisation, par un traitement thermique ou par radiations, qui risque de provoquer la rupture de la membrane sous l'effet de la dilatation du liquide qui la remplit.

En l'absence d'autres techniques de stérilisation, l'invention vise à éliminer cette difficulté par le remplacement de tels volumes individuels remplis de liquide par des particules de matériau sec possédant la particularité d'absorber l'eau en se gonflant jusqu'à constituer chacune une particule souple ayant la consistance d'un gel et un volume de l'ordre de plusieurs fois celui de la particule sèche de départ.

De tels volumes individuels ne possédant pas de membrane périphérique, l'inconvénient précité est éliminé ipso facto.

Il existe de nombreux matériaux susceptibles de gonfler au contact de l'eau, et l'invention consiste dans le choix d'une catégorie de ces matériaux possédant d'autres propriétés additionnelles les rendant aptes à une telle application.

Ces matières sont les associations silicones-hydrogels.

Les hydrogels sont des composés bien connus pour d'autres applications, par exemple comme véhicules d'eau dans l'agriculture et comme absorbants dans les articles d'hygiène.

On ne peut cependant pas les utiliser tels quels dans la présente application car, compte tenu de leur structure chimique ils tendraient à se lier les uns aux autres dans l'enveloppe de l'implant, en aboutissant à une masse trop rigide.

C'est pourquoi, selon l'invention, on associe de tels hydrogels à un élastomère de silicone pour créer des granules secs destinés à remplir l'enveloppe extérieure de l'implant de manière à permettre la stérilisation de ce dernier, par les moyens connus, mais sans les inconvénients précités, de l'eau ou du sérum physiologique étant ensuite injecté dans l'implant après sa mise en place dans le décollement sous-mammaire, déterminant alors le gonflement desdits granules jusqu'au volume définitif souhaité.

L'association hydrogel/silicone selon l'invention peut se faire de diverses manières, rentrant toutes dans le cadre de l'invention.

Selon une première réalisation, chaque granule de silicone est enrobé d'hydrogel, selon une technique connue pour la construction de cathéters intravasculaires (cf. Vladimir STOY et Charles KLIMENT dans "Hydrogels : Speciality Plastics for Biomedical and Pharmaceutical Applications").

Selon une seconde réalisation, l'hydrogel est associé aux granules de silicone non pas par encapsulation mais par capillarité, le granule de silicone étant poreux et imprégné d'hydrogel. Dans ce cas, à l'hydratation, c'est tout l'ensemble qui gonfle. Après une telle hydratation, l'hydrogel peut être exprimé par pression sur la sphérule gonflée, mais y retourne par capillarité quand la pression est relâchée.

Selon une troisième réalisation, qui constitue la variante préférée de l'invention, l'association hydrogel/silicone, au lieu d'être mécanique comme précédemment, est de nature chimique, par copolymérisation entre un hydrogel et un polysiloxane.

Dans ce dernier cas, le composant hydrogel du copolymère peut être biodégradable ou non.

Quand il est biodégradable, il est détruit en cas de rupture de l'implant. Il convient alors que les produits de dégradation ne soient pas toxiques pour l'organisme.

Quand il n'est pas biodégradable, il demeure lié à l'élastomère de silicone et est enlevé au moment d'une ré-intervention en cas de rupture de l'implant.

Dans la plupart des cas, on se situera entre ces deux extrêmes.

De tels copolymères sont donc des hydrogels hybrides qui, subdivisés à l'état de particules telles que des granules ou des microbilles, sont utilisés, selon l'invention, pour le remplissage de l'enveloppe usuelle en silicone d'un implant tel qu'un implant mammaire.

Du fait que ces particules sont sèches, elles sont faciles à stériliser et n'offrent donc pas l'inconvénient précité.

Une fois l'enveloppe remplie de la quantité voulue de ces particules sèches, elle est mise en place dans le décollement sous-mammaire et on y injecte de l'eau stérile au moyen d'une aiguille au travers un "patch" de sécurité, c'est à dire d'une zone de l'enveloppe extérieure de l'implant formée par une membrane ayant la propriété de se refermer d'elle-même après avoir été perforée par une aiguille.

La quantité d'eau injectée doit être légèrement supérieure à celle que l'hydrogel peut fixer, dans le but d'atteindre le volume souhaité pour l'implant, et le degré maximum d'hydratation de l'hydrogel et qui dépend du nombre de particules et de leur volume à l'état humide, fonction de leur taux d'absorption.

Au gonflement, l'ensemble desdites particules ne vont pas se fusionner mais demeurent mobiles les unes par rapport aux autres, de sorte que, même en cas de rupture de l'enveloppe extérieure de l'implant, il n'y a pas de risque de fuite de l'eau hors de ladite enveloppe bien que les particules hydratées puissent se retrouver libres dans la sphère cicatricielle périprothétique ; lors du changement éventuel d'un implant rompu, il est facile au chirurgien de récupérer les particules d'hydrogel gonflé, par simple aspiration.

A titre d'exemples d'hydrogels connus en eux-mêmes pouvant être copolymérisés avec des silicones pour constituer les matériaux formant les particules sèches selon l'invention, on peut citer notamment les polyacrylamides réticulées par divers monomères et décrites dans diverses publications (C.A., 69, N° 17, 1968 et C.A., 74, N° 3, 1971).

Pour ce qui concerne la membrane ou enveloppe extérieure de l'implant, diverses variantes sont également possibles dans le cadre de la présente invention.

Selon une première réalisation, cette membrane peut être en élastomère de silicone, lisse ou rugueux. Elle doit alors comporter une zone épaisse, ou "patch", permettant, par perforation temporaire au moyen d'une aiguille, d'introduire la quantité d'eau ou de sérum physiologique provoquant le gonflement des granules jusqu'au volume désiré.

Ce remplissage peut également se faire par l'intermédiaire d'une valve et d'un tube remplisseur qui est enlevé en fin d'intervention ou même ultérieurement.

Selon une seconde réalisation, cette membrane peut également être réalisée en copolymère silicone/hydrogel ayant la propriété de se refermer spontanément après avoir été perforée (auto-cicatrisante). L'avantage d'une telle membrane est de permettre une mise en place aisée de l'implant à l'état sec, puis un gonflement graduel en période post-opératoire, par remplissage effectué par des ponctions au hasard à travers la peau.

Cette opération est particulièrement importante dans les indications de reconstruction immédiate après ablation du sein.

L'implant selon l'invention évite, entre autres, deux inconvénients majeurs des implants actuellement connus :
- il élimine la présence de gel de silicone dont les chaînes courtes peuvent migrer hors de l'implant vers le tissu périprothétique, ce qui représente un facteur important dans la genèse de la capsule contractile périprothétique ;
- il empêche le dégonflement partiel ou total des implants gonflables au sérum physiologique, même en cas de ponction dans l'implant ou de rupture de l'enveloppe extérieure.

Par ailleurs, il possède l'avantage que l'association silicone/hydrogel confère à l'élément de base du remplissage de l'enveloppe, à savoir un granule de silicone hydraté par l'hydrogel, une architecture stable, facile à enlever par simple aspiration en cas de rupture de l'enveloppe.

Un tel élément de base, dont le volume, à l'état gonflé au maximum, se situe de préférence entre 1 et 5 cm³ , peut être facilement détecté par échographie ou radiographie, en particulier quand il est hors de l'enveloppe de l'implant, ce qui permet ainsi de dépister les ruptures de ladite enveloppe.

Bien entendu, bien que l'application le plus important des implant selon l'invention soit la mammoplastie, de tels implants pourront être adaptés à la restructuration d'autres zones du corps humain, telle que les mollets, le menton, le cuir chevelu, en bénéficiant des mêmes avantages.

On pourra également utiliser les mêmes granules d'hydrogel-hybride pour traitement réparateur des rides du visage. Dans cette application particulière, les particules sèches d'hydrogel hybride sont infiltrées directement dans les rides, leur gonflement étant obtenu par injection d'eau ou de sérum physiologique à travers la peau, qui joue alors le rôle de l'enveloppe extérieure.

## Revendications

1. Implant prothétique, destiné à la reconstruction ou la modification en forme et/ou en dimensions des zones du corps humain telle que les seins, les mollets, le menton, le cuir chevelu, et analogues, caractérisé en ce qu'il consiste en une enveloppe extérieure remplie de particules sèches d'une association hydrogel/élastomère de silicone, dont le gonflement est provoqué après mise en place par injection d'eau ou de sérum physiologique.

2. Implant selon la revendication 1, caractérisé en ce que chaque particule sèche est un granule de silicone enrobé d'hydrogel.

3. Implant selon la revendication 1, caractérisé en ce que chaque particule sèche est une sphérule poreuse imprégnée d'hydrogel.

4. Implant selon la revendication 1, caractérisé en ce que chaque particule sèche est un granule d'un hydrogel hybride obtenu par copolymérisation entre un hydrogel et un polysiloxane.

5. Implant selon l'une quelconque des revendications 1 à 4, caractérisé en ce que l'hydrogel est biodégradable, ses produits de dégradation étant non-toxiques pour l'organisme humain.

6. Implant selon l'une quelconque des revendications 1 à 4, caractérisé en ce que l'hydrogel n'étant pas bio-dégradable, il demeure lié au polysiloxane, et en cas de rupture de l'implant, il est extrait au moment d'une ré-intervention.

7. Implant selon l'une quelconque des revendications 1 à 6, caractérisé en ce que l'enveloppe extérieure est une membrane en élastomère de silicone comportant une zone permettant la traversée temporaire d'une aiguille de remplissage d'eau ou de sérum physiologique.

8. Implant selon l'une quelconque des revendications 1 à 6, caractérisé en ce que l'enveloppe extérieure est une membrane auto-cicatrisante en copolymère hydrogel/silicone.

## Claims

1. Prosthetic implant intended for reconstructing or modifying the shape and/or dimensions of parts of the human body, such as the breasts, calves, chin, scalp etc., characterised by the fact that it consists of an outer envelope filled with dry particles of a hydrogel/silicone elastomer combination, whose dilation is provoked, after installation, by injecting water or physiologocal serum.

2. Implant according to claim 1, characterised by the fact that each dry particle is a granule of silicone coated with hydrogel.

3. Implant according to claim 1, characterised by the fact that each dry particle is a porous sphere impregnated with hydrogel.

4. Implant according to claim 1, characterised by the fact that each dry particle is a granule of a hybrid hydrogel obtained by copolymerisation between a hydrogel and a polysiloxane.

5. Implant according to any of claims 1 to 4, characterised by the fact that the hydrogel is biodegradable, its degradation products being non-toxic for humans.

6. Implant according to any of claims 1 to 4, characterised by the fact that the hydrogel being non-biodegradable, it remains bonded to the polysiloxane, and in the event of the rupture of the implant, it is extracted during a second operation.

7. Implant according to any of claims 1 to 6, characterised by the fact that the outer envelope is a membrane of silicone elastomer with a zone allowing temporary insertion of a needle for filling with water or physiological serum.

8. Implant according to any of claims 1 to 6, characterised by the fact that the outer envelope is a self-sealing membrane of a hydrogel/silicone copolymer.

## Patentansprüche

1. Prothetisches Implantat, bestimmt zur Rekonstruktion oder Modifikation der Form und/oder der Dimensionen von menschlichen Körperteilen wie der Brüste, der Waden, des Kinns, der Kopfhaut oder ähnlichen, dadurch gekennzeichnet, daß es aus einer äußeren Hülle besteht, die gefüllt ist mit Trockenpartikeln eines Hydrogel/Silikonelastomer-Assoziats, dessen Aufquellen nach der Plazierung an Ort und Stelle durch Injektion von Wasser oder physiologischem Serum ausgelöst wird.

2. Implantat nach Anspruch 1, dadurch gekenzeichnet, daß jede Trockenpartikel ein mit Hydrogel umhülltes Silikonkörnchen ist.

3. Implantat nach Anspruch 1, dadurch gekennzeichnet, daß jede Trockenpartikel ein mit Hydrogel imprägniertes poröses Kügelchen ist.

4. Implantat nach Anspruch 1, dadurch gekennzeichnet, daß jede Trockenpartikel ein durch Copolymerisation von einem Hydrogel und einem Polysiloxan erhaltenes Hybrid-Hydrogel-Körnchen ist.

5. Implantat nach einem der Ansprüche 1 bis 4, dadurch gekennzeichnet, daß das Hydrogel biologisch abbaubar ist, wobei seine Abbauprodukte für den menschlichen Organismus nicht toxisch sind.

6. Implantat nach einem der Ansprüche 1 bis 4, dadurch gekennzeichnet, daß das Hydrogel, falls es nicht biologisch abbaubar ist, an Polysiloxan gebunden bleibt, und im Fall des Reißens des Implantats bei einem erneuten Eingriff entnommen wird.

7. Implantat nach einem der Ansprüche 1 bis 6, dadurch gekennzeichnet, daß die äußere Hülle eine Membran aus einem Silikon-Elastomer ist, umfassend eine Zone, die die zeitweise Durchdringung mit einer Nadel für das Nachfüllen mit Wasser oder physiologischem Serum ermöglicht.

8. Implantat nach einem der Ansprüche 1 bis 6, dadurch gekennzeichnet, daß die äußere Hülle eine selbstvernarbende Membran aus einem Hydrogel/Silikon-Copolymer ist.
